# EUROPÄISCHE PATENTSCHRIFT

(11) **EP 1 165 482 B1**
(45) Veröffentlichungstag und Bekanntmachung des Hinweises auf die Patenterteilung: **15.10.2003**
(21) Anmeldenummer: 00903608.8
(22) Anmeldetag: 22.01.2000
(51) Int. Cl.: C07C 51/41, C07C 53/126, C08K 5/098

(54) **VERFAHREN ZUR HERSTELLUNG ÜBERBASISCHER ZINKSEIFEN**
METHOD FOR PRODUCING OVERBASED ZINC SOAPS
PROCEDE DE PRODUCTION DE SAVONS DE ZINC HYPERBASIQUES

(30) Priorität: 03.02.1999 DE 19904139
(43) Veröffentlichungstag der Anmeldung: 02.01.2002
(73) Patentinhaber: Cognis Deutschland GmbH & Co. KG, 40589 Düsseldorf (DE)
(72) Erfinder: DAUTE, Peter, D-27616 Beverstedt (DE); KLAMANN, Jörg-Dieter, D-27574 Bremerhaven (DE); WEDL, Peter, D-27568 Bremerhaven (DE); PICARD, Ralf, D-27572 Bremerhaven (DE)
(86) Internationale Anmeldenummer: EP0000466
(87) Internationale Veröffentlichungsnummer: WO00046173

(56) Entgegenhaltungen:
- EP-A- 0 234 149
- WO-A-95/34524
- GB-A- 1 142 195
- GB-A- 2 110 697

## Beschreibung

### Gebiet der Erfindung

Die Erfindung betrifft ein Verfahren zur Herstellung überbasischer Zinkseifen, wobei man basisches Zinkcarbonat mit organischen Carbonsäuren in Abwesenheit organischer Lösungsmittel direkt miteinander umsetzt.

### Stand der Technik

Metallseifen werden üblicherweise durch direkte Umsetzung von Metalloxiden oder Metallhydroxiden mit den entsprechenden organischen Carbonsäuren hergestellt oder durch doppelte Umsetzung, bei der Metallseifen aus heißer wäßriger oder wäßrig-alkoholischer Seifenlösung durch Zusatz von Salzlösungen der betreffenden Metalle ausgefällt werden (siehe beispielsweise **Ullmann's Encyklopädie der Technischen Chemie, 4. Auflage, Band 21, Seite 224)**.

Aus **EP-B-0 234 149** ist ein Verfahren zur Herstellung überbasischer Caiciumseifen bekannt, bei welchem man unter Rühren ein Oxid und/oder ein Hydroxid des Calciums mit Kohlendioxid, das man in das Reaktionsmilieu einbläst, und mindestens einer organischen Carbonsäure in Gegenwart mindestens eines Promotors und mindestens eines Katalysators reagieren läßt und das gebildete Wasser entfernt, wobei die Reaktion in mindestens einem organischen Lösungsmittel bei Temperaturen zwischen 80 und 120°C durchgeführt wird, die organische Carbonsäure 7-13. C-Atome aufweist, deren Gehalt an linearen Säuren 40 Gew.% oder weniger beträgt, deren Gehalt an am C-2 verzweigten Säuren 20 Gew.% oder weniger beträgt und deren Gehalt an C-3 und/oder C-n verzweigten Säuren 40 Gew.% oder mehr beträgt und daß man am Ende der Reaktion das organische Lösungsmittel durch ein Öl oder eine Mischung aus Ölen ersetzt. Der Promotor soll dabei zur Fixierung des Kohlendioxids dienen.

Aus **WO 95/34524** ist ein Verfahren zur Herstellung überbasischer Zinkseifen bekannt, wobei man ein Zinkoxid und/oder -hydroxid unter Rühren mit gasförmigem Kohlendioxid und wenigstens einer organischen Carbönsäure umsetzt. Die Umsetzung geschieht in Gegenwart mindestens eines Promotors, der die Fixierung von Kohlendioxid erleichtem soll. Darüber hinaus wird das während der Reaktion gebildete Wasser fortlaufend entfernt. Vorzugsweise wird die Reaktion bei Temperaturen zwischen 80 und 120°C und in Gegenwart mindestens eines organischen Lösungsmittels durchgeführt.

### Beschreibung der Erfindung

Es wurde nun gefunden, daß sich überbasische Zinkseifen in einfacher Weise dadurch herstellen lassen, daß man Zinkhydroxycarbonat (basisches Zinkcarbonat) mit organischen Carbonsäuren direkt umsetzt. Die Umsetzung wird dabei bei Temperaturen im Bereich von 100 bis 200°C durchgeführt.

Gegenstand der vorliegenden Erfindung ist ein Verfahren zur Herstellung überbasischer Zinkseifen, wobei man Zinkhydroxycarbonat mit organischen Carbonsäuren mit 6 bis 22 C-Atomen bei Temperaturen im Bereich von 100 bis 200°C in Abwesenheit organischer Lösungsmittel umsetzt und das entstehende Reaktionswasser kontinuierlich abdestilliert.

Das erfindungsgemäße Verfahren läßt sich in wesentlich einfacherer Weise durchführen als die aus dem Stand der Technik bekannten Verfahren. Insbesondere wird vermieden, daß im Zuge der Herstellung der überbasischen Zinkseifen mit gasförmigem Kohlendioxid gearbeitet werden muß. Auch der Einsatz spezieller Promotoren, die eine Bindung von Kohlendioxid erleichtern sollen, entfällt. Darüber hinaus wird die Umsetzung erfindungsgemäß in einer Schmelze von Zinkhydroxycarbonat und organischen Carbonsäuren durchgeführt. Dementsprechend entfällt auch der Umgang mit einem Lösungsmittel sowie dessen anschließende Entfernung.

Basisches Zinkcarbonat entsteht, wenn neutrales Zinkcarbonat, ZnCO₃, mit Wasser reagiert. Basisches Zinkcarbonat kommt in der Natur als Zinkblüte der Zusammensetzung Zn₅[(OH)₃(CO₃)]₂ vor, ist jedoch auch kommerziell erhältlich.

Wie bereits gesagt wird das erfindungsgemäße Verfahren bei Temperaturen im Bereich von 100 bis 200°C durchgeführt. In einer bevorzugten Ausführungsform arbeitet man im Temperaturbereich von 110 bis 160°C und insbesondere im Temperaturbereich von 130 bis 150°C.

Wie bereits gesagt werden im Zuge des erfindungsgemäßen Verfahrens organische Carbonsäuren mit 6 bis 22 C-Atomen eingesetzt. Diese Säuren können gesättigt oder ungesättigt, geradkettig oder verzweigt sein. Sie können entweder einzeln oder als Gemisch von zwei oder mehreren Fettsäuren eingesetzt werden. Beispiele für geeignete organische Carbonsäuren mit 6 bis 22 C-Atomen sind Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Heptadecansäure, Stearinsäure, Nonadecansäure, Arachinsäure, Heneicosansäure, Behensäure, 10-Undecensäure, Lauroleinsäure, Myristoleinsäure, Palmitoleinsäure, Ölsäure, Petroselinsäure, Elaidinsäure, Ricinolsäure, 12-Hydroxystearinsäure, 9,10-Dihydroxystearinsäure, 9,10,11,12-Tetrahydroxystearinsäure, Linolsäure, Linolaidinsäure, Linolensäure, Eläostearinsäure, Gadoleinsäure, Arachidonsäure, Erucasäure, Brassidinsäure, Clupanodonsäure.

In einer Ausführungsform setzt man ein oder mehrere organische Carbonsäuren mit 6 bis 22 C-Atomen ein, wobei der Gehalt an linearen Carbonsäuren mindestens 90 Gew.% - bezogen auf die Gesamtmenge der eingesetzten Carbonsäuren - beträgt. Diese linearen Carbonsäuren sind dabei vorzugsweise zu mindestens 60 Gew.% gesättigt.

In einer weiteren Ausführungsform setzt man ein oder mehrere organische Carbonsäuren mit 6 bis 22 C-Atomen ein, wobei der Gehalt an am C-2 verzweigten Säuren mindestens 90 Gew.% - bezogen auf die Gesamtmenge der eingesetzten Carbonsäuren - beträgt. Vorzugsweise setzt man hier 2-Ethyl-hexansäure ein.

Hinsichtlich des Verhältnisses, in dem die beiden Reaktionskomponenten im Zuge des erfindungsgemäßen Verfahrens, nämlich einerseits das Zinkhydroxycarbonat und andererseits die organischen Carbonsäuren miteinander umgesetzt werden, bestehen an sich keinerlei Beschränkungen. Vorzugsweise setzt man die beiden Komponenten jedoch in einer Menge ein, daß das Äquivalentverhältnis OH/COOH im Bereich von 0,5:1 bis 10:1 und insbesondere im Bereich von 0,5:1 bis 2:1 liegt. Besonders bevorzugt stellt man ein Äquivalentverhättnis von etwa 1:1 ein.

Der Ausdruck Äquivalentverhältnis" ist dem Fachmann geläufig. Der grundlegende Gedanke hinsichtlich des Begriffs der Äquivalenz ist der, daß man für jede an einer Reaktion beteiligten Substanz die an der angestrebten Reaktion beteiligten reaktiven Gruppen betrachtet. Durch die Angabe eines Äquivalentverhältnisses drückt man dann aus, in welchem Zahlenverhältnis die Gesamtheit der reaktiven Gruppen der eingesetzten Verbindungen zueinander stehen. Im vorliegenden Fall bedeutet dies folgendes: Basisches Zinkcarbonat wird durch die Formel [ZnCO₃]₂x[Zn(OH)₂]₃ repräsentiert. Die organischen Carbonsäuren werden durch die Formel C₅₋₂₁-COOH repräsentiert, wobei C₅₋₂₁ für eine Alkylrest mit 5 bis 21 C-Atomen steht. Ein Äquivalentverhältnis OH/COOH von 1:1 bedeutet dann, daß man das basische Zinkcarbonat mit der organische Carbonsäure in einem Mengenverhältnis umsetzt, daß im eingesetzten Zinkhydroxycarbonat ebenso viele OH-Gruppen vorhanden sind wie in der eingesetzten organischen Carbonsäure COOH-Gruppen. Da basisches Zinkcarbonat gemäß der genannten Formel sechs OH-Gruppen enthält, die organische Carbonsäure jedoch nur eine COOH-Gruppe, müssen basisches Zinkcarbonat und organische Carbonsäure in einem Molverhältnis von 1:6 eingesetzt werden.

Die nach dem erfindungsgemäßen Verfahren erhältlichen überbasischen Zinkseifen eignen sich als Additive für die Verarbeitung von Kunststoffen und insbesondere zum Stabilisieren von halogenhaltigen Kunststoffen gegen thermischen und/oder photochemischen Abbau.

Die nach dem erfindungsgemäßen Verfahren zugänglichen überbasischen Zinkseifen eignen sich als Additive zur Stabilisierung von halogenhaltigen organischen Kunststoffen, insbesondere Polyvinylchlorid (PVC).

### Beispiele

### A. Herstellung überbasischer Zinkseifen

### Beispiel 1: Herstellung von Zink-Carbonat-Stearat

162 g (0,6 Mol) technische Stearinsäure mit einer Säurezahl von 208 wurden unter Rühren auf 140°C erhitzt und innerhalb von 10 Minuten mit 54,9 g (0,1 Mol) basischem Zinkcarbonat der Formel [ZnCO₃]₂ x [Zn(OH)₂]₃ (Firma Fluka) versetzt. Unter Schäumen entwich das gebildete Reaktionswasser innerhalb von 30 Minuten. Die leicht weißliche Schmelze wurde weitere 1,5 Stunden auf 130 bis 140°C erwärmt und anschließend auf 20°C abgekühlt.

Man erhielt eine weiße, wachsartige Masse. Die analytischen Daten dieser Masse waren wie folgt: Zinkgehalt = 16,4 Gew.%; Schmelzpunkt = 118°C; Säurezahl = 9,8.

### Beispiel 2: Herstellung von Zink-Carbonat-Ethylhexanoat

Analog zu Beispiel 1, wobei jedoch anstelle der Stearinsäure 0,6 Mol 2-Ethylhexansäure eingesetzt wurden. Die pastöse Masse wies einen Zinkgehalt von 23,7 Gew.% auf.

### Beispiel 3: Herstellung von Zink-Carbonat-Octoat

Wie Beispiel 1, jedoch wurden anstelle der Stearinsäure 0,6 Mol n-Octansäure eingesetzt. Der Schmelzpunkt der erhaltenen Masse wurde zu 97°C bestimmt. Der Zinkgehalt lag bei 25,3 Gew.%.

### Beispiel 4: Herstellung von Zink-Carbonat-Laurat

Wie Beispiel 1, jedoch wurden anstelle von Stearinsäuren 0,6 Mol Laurinsäure eingesetzt. Der Schmelzpunkt der erhaltenen Masse lag bei 113°C. Der Zinkgehalt wurde zu 20,8 Gew.% bestimmt.

### B. Anwendungstechnische Prüfungen

Die gemäß den obengenannten Beispielen 1 bis 4 hergestellten Substanzen wurden hinsichtlich ihrer Fähigkeit geprüft, die Farbstabilität von Polyvinylchlorid (PVC) zu verbessern. Hierzu wurde die unten erläuterte Methode der Farbmessung durchgeführt, wobei spezielle Prüfkörper bei 190°C temperiert wurden und der zeitliche Verlauf der Farbwerte registriert wurde.

Als Prüfkörper dienten Walzfelle, aus denen Teststreifen herausgestanzt wurden. Der Herstellung der Walzfelle lag folgende Testrezeptur zugrunde:

| | |
|---|---|
| PVC (Solvic 271 GC; Firma Solvay) | 100,0 Gewichtsteile |
| Dioctylphthalat | 40,0 Gewichtsteile |
| Edenol D81 (epoxidiertes Sojaöl; Firma Henkel KGaA) | 4,0 Gewichtsteile |
| Kreide | 30,0 Gewichtsteile |
| Titandioxid | 3,0 Gewichtsteile |
| Zink-freier Stabilisator | 1,96 Gewichtsteile |
| Prüfsubstanz^{a)} | x Gewichtsteile |

| | |
|---|---|
| a) Prüfsubstanz = die gemäß den obengenannten Beispielen 1 bis 4 hergestellten Substanzen | |

Die Prüfkörper wurden hergestellt, indem man die PVC-Hartmasse und die genannten Zusatzstoffe auf einem Laborwalzwerk 5 Minuten bei 170°C homogenisierte und plastifizierte. Aus den so hergestellten etwa 0,5 mm dikken Walzfellen wurden Teststreifen von 15 mm Breite herausgeschnitten.

Unmittelbar nach der Herstellung der Walzfelle wurde deren Farbe, die sogenannte Anfangsfarbe (t = 0) bestimmt. Hierzu wurde die dem Fachmann bekannte L*, a*, b*-Methode (vergleiche DIN 6174) herangezogen. Der b*-Wert gibt dabei die Lage auf der Blau/Gelb-Achse an. Üblicherweise wird der b*-Wert auch Gelbwert genannt. Bei den Messungen kam ein handelsübliches Gerät mit der Bezeichnung "Micro-Color" (Firma Dr. Lange) zum Einsatz. Die Anfangsfarben sind in der Tabelle 1 zusammengestellt. Tabelle 1 enthält auch Angaben darüber, in welcher Menge die Prüfsubstanz in der obengenannten Testrezeptur enthalten war. Die Teststreifen wurden anschließend bei 190°C in einem Thermoofen getempert, und dabei in Abständen von jeweils 15 Minuten jeweils kurz aus dem Ofen herausgefahren, um den jeweiligen b*-Wert zu bestimmen. Die entsprechenden b*-Werte, die nach 15, 30, 45 und 60 Minuten gemessen wurden, sind ebenfalls in Tabelle 1 zusammengestellt.

**Tabelle 1**

| Prüfsubstanz | X^{b)} | b*/0^{c)} | b*/15^{d)} | b*/30^{e)} | b*/45^{f)} | b*/60^{g)} |
|---|---|---|---|---|---|---|
| Beispiel 1 | 0,84 | 6,4 | 9,4 | 11,4 | 13,4 | 17,7 |
| Beispiel 2 | 0,58 | 6,2 | 9,5 | 11,1 | 14,6 | 19,5 |
| Beispiel 3 | 0,55 | 6,3 | 9,4 | 11,7 | 13,9 | 20,4 |
| Beispiel 4 | 0,60 | 6,3 | 9,4 | 11,4 | 14,2 | 20,9 |

| | | | | | | |
|---|---|---|---|---|---|---|
| b) X = Anteil der Prüfsubstanz in Gewichtsteilen in der oben genannten Testrezeptur (die Verbindungen gemäß den Beispielen 1 bis 4 wurden Zn-gleich eingesetzt). | | | | | | |
| c) b*/0 = Anfangsfarbe (Gelbwert b* nach 0 Minuten Temperaturbelastung) | | | | | | |
| d) b*/15 = Gelbwert b* nach 15 Minuten Temperaturbelastung bei 190°C | | | | | | |
| e) b*/30 = Gelbwert b* nach 30 Minuten Temperaturbelastung bei 190°C | | | | | | |
| f) b*/45 = Gelbwert b* nach 45 Minuten Temperaturbelastung bei 190°C | | | | | | |
| g) b*/60 = Gelbwert b* nach 60 Minuten Temperaturbelastung bei 190°C | | | | | | |

## Patentansprüche

1. Verfahren zur Herstellung überbasischer Zinkseifen, wobei man basisches Zinkcarbonat mit organischen Carbonsäuren mit 6 bis 22 C-Atomen bei Temperaturen im Bereich von 100 bis 200°C in Abwesenheit organischer Lösungsmittel umsetzt und das entstehende Reaktionswasser kontinuierlich abdestilliert.

2. Verfahren nach Anspruch 1, wobei man die Temperatur im Bereich von 130° bis 150° C einstellt.

3. Verfahren nach Anspruch 1 oder 2, wobei der Gehalt der organischen Carbonsäuren an linearen Carbonsäuren mindestens 90 Gew.-% - bezogen auf die Gesamtmenge der eingesetzten Carbonsäuren - beträgt.

4. Verfahren nach Anspruch 1 oder 2, wobei der Gehalt der organischen Carbonsäuren an am C-2 verzweigten Säuren mindestens 90 Gew.-% - bezogen auf die Gesamtmenge der eingesetzten Carbonsäuren - beträgt.

5. Verfahren nach Anspruch 4, wobei man als organische Carbonsäuren 2-Ethyl-hexansäure einsetzt.

6. Verfahren nach einem der Ansprüche 1 bis 5, wobei man die beiden Reaktionskomponenten (basisches Zinkcarbonat und organische Carbonsäuren) in einer Menge einsetzt, dass das Äquivalentverhältnis OH/COOH im Bereich von 0,5:1 bis 2:1 liegt.

## Claims

1. A process for the production of superbasic zinc soaps, **characterized in that** basic zinc carbonate is reacted with organic carboxylic acids containing 6 to 22 carbon atoms at temperatures of 100 to 200°C in the absence of organic solvents and the water of reaction formed is continuously distilled off.

2. A process as claimed in claim 1, **characterized in that** the temperature is adjusted to a value in the range from 130°C to 150°C.

3. A process as claimed in claim 1 or 2, **characterized in that** the content of linear carboxylic acids in the organic carboxylic acids is at least 90% by weight, based on the total quantity of the carboxylic acids used.

4. A process as claimed in claim 1 or 2, **characterized in that** the content of acids branched at C-2 in the organic carboxylic acids is at least 90% by weight, based on the total quantity of carboxylic acids used.

5. A process as claimed in claim 4, **characterized in that** 2-ethyl hexanoic acid is used as the organic carboxylic acid.

6. A process as claimed in any of claims 1 to 5, **characterized in that** the two reaction components (basic zinc carbonate and organic carboxylic acids) are used in such a quantity that the equivalent OH:COOH ratio is in the range from 0.5:1 to 2:1.

## Revendications

1. Procédé de production de savons de zinc hyper basiques,
dans lequel
on fait réagir du carbonate de zinc basique avec des acides carboxyliques organiques ayant de 6 à 22 atomes de carbone, à des températures dans la plage de 100 à 200°C en l'absence de solvant organique et on sépare par distillation en continu, l'eau de réaction qui s'est formée.

2. Procédé selon la revendication 1,
dans lequel
on ajuste la température dans une plage allant de 130 à 150°C.

3. Procédé selon la revendication 1 ou la revendication 2,
dans lequel
les acides carboxyliques organiques ont une teneur en acides carboxyliques linéaires d'au moins 90 % en poids, rapporté à la quantité totale des acides carboxyliques mis en oeuvre.

4. Procédé selon la revendication 1 ou la revendication 2,
dans lequel
les acides carboxyliques organiques ont une teneur en acides ramifiés en C₂ d'au moins 90 % en poids, rapporté à la quantité totale des acides carboxyliques mis en oeuvre.

5. Procédé selon la revendication 4,
dans lequel
comme acide carboxylique organique on met en oeuvre de l'acide 2-éthyl hexanoïque.

6. Procédé selon l'une des revendications 1 à 5,
dans lequel
on met en oeuvre les deux composants réactionnels (carbonate de zinc basique et acide organique carboxylique) en une quantité telle que le rapport en équivalents OH / COOH se situe dans la zone de 0,5 : 1 à 2 : 1.
